# EUROPÄISCHE PATENTSCHRIFT

(11) **EP 4 302 737 B1**
(45) Veröffentlichungstag und Bekanntmachung des Hinweises auf die Patenterteilung: **03.09.2025**
(21) Anmeldenummer: 22183621.6
(22) Anmeldetag: 07.07.2022
(51) Int. Cl.: A61F 13/08, A41B 1/00

(54) **BEINBEKLEIDUNGSSTÜCK ZUR MASSAGE UND KOMPRESSION**
LEG GARMENT FOR MASSAGE AND COMPRESSION
PIÈCE DE BONNETERIE POUR LE MASSAGE ET LA COMPRESSION

(43) Veröffentlichungstag der Anmeldung: 10.01.2024
(73) Patentinhaber: Edelweiss Basics GmbH & Co. KG, 83098 Brannenburg (DE)
(72) Erfinder: Weber-Unger, Georg, 6330 Kufstein (AT)
(74) Vertreter: Kador & Partner Part mbB

(56) Entgegenhaltungen:
- US-A1- 2018 279 694
- US-B2- 7 945 970
- US-B2- 8 172 782

## Beschreibung

Die vorliegende Erfindung betrifft ein Beinbekleidungsstück zur Massage und Kompression von Körperbereichen einer Trägerin oder eines Trägers geeignet.

Kleidungs- und Kompressionselemente kann man in US2018279694, US7945970 und US8172782 finden. Unter Kompressionsbekleidung wird üblicherweise spezielle Sportkleidung verstanden, die sehr eng am Körper und auf den Körper auswirkt. Die Kompressionskleidung übt Druck auf die Muskulatur und die Venen aus und sorgt dafür, dass das Blut besser und schneller fließt. Durch diesen besseren und schnelleren Blutfluss wird mehr Sauerstoff in die Muskulatur gepumpt. Kompression und/oder Massage wird unter anderem auch als therapeutische Maßnahme bei Lymphödemen angewandt, zumeist mittels eines Kompressionsstrumpfes.

Ein Nachteil des Standes der Technik ist, dass Kompressionsbekleidung einen einheitlichen Druck und somit eine einheitliche Kompression auf den Körper bzw. die Körperpartien des Trägers bzw. der Trägerin bewirken. Darunter kann jedoch der Tragekomfort leiden, da auch Druck auf Körperstellen ausgeübt wird an jenen eine Kompression als unangenehm empfunden wird.

Auch ist ein Nachteil, dass Kompressionsbekleidung aufgrund des engen Anliegens auf der Haut üblicherweise zu vermehrten Schwitzen des Trägers oder der Trägerin führt. Der Grund ist, dass durch das enge Anliegen der Kompressionsbekleidung die Belüftung der Haut eingeschränkt wird.

Die Erfindung hat die Aufgabe ein Beinbekleidungsstück bereitzustellen, welches die oben genannten Nachteile vermindert oder gar beseitigt. Es ist daher eine Aufgabe der Erfindung ein Bekleidungsstück für den Beinbereich bereitzustellen, welches sowohl die nötige Kompression und zugleich auch einen hohen Tragekomfort für den Träger oder die Trägerin aufweist.

Es ist weiter eine Aufgabe der Erfindung ein Beinbekleidungsstück bereitzustellen, welches zugleich eine gute Massagewirkung hat und stützend wirkt.

All die gestellten Aufgaben werden durch das erfindungsgemäße Beinbekleidungsstück gelöst.

Der Erfindung liegt die Erkenntnis zugrunde, dass die oben gestellten Aufgaben durch ein Beinbekleidungsstück gelöst werden können, wobei das Beinbekleidungsstück drei oder mehrere unterschiedliche Stoffmaterialen umfasst oder daraus besteht, insbesondere drei oder mehrere funktional unterschiedliche Stoffmaterialen oder daraus besteht, insbesondere drei funktional unterschiedliche Stoffmaterialen oder daraus besteht, nämlich ein erstes Stoffmaterial, ein zweites Stoffmaterial und ein drittes Stoffmaterial. Funktional unterschiedlich bedeutet, dass sich die jeweiligen Stoffmaterialien in ihren Kompressionseigenschaften, ihren Dehnungseigenschaften, ihren Quadratmetergewicht, ihrer Luftdurchlässigkeit, ihrer Oberflächenstruktur, ihren Fasereigenschaften und/oder ihren Garneigenschaften voneinander unterscheiden. Die drei oder mehr Stoffmaterialen sind also unterschiedlich.

Die Erfindung stellt daher ein Beinbekleidungsstück, vorzugsweise aus drei oder mehr unterschiedlichen Stoffmaterialien, umfassend oder bestehend aus einem Bund, einem Vorderteil, einem Rückteil, zwei Seitenteile, zwei Kniekehlenteile sowie zwei Wadenteile bereit, wobei das Vorderteil, die Seitenteile und das Rückteil jeweils mit dem Bund verbunden sind, dadurch gekennzeichnet, dass die zwei Seitenteile, das Rückteil und die zwei Wadenteile jeweils ein erstes Stoffmaterial zur Massage und Kompression von Beinbereichen einer Trägerin umfasst, dass das Vorderteil und die Kniekehlenteile jeweils ein zweites Stoffmaterial umfasst, und dass der Bund ein drittes Stoffmaterial umfasst,
wobei das erste Stoffmaterial eine höhere Kompression aufweist als das zweite Stoffmaterial.

Das erfindungsgemäße Beinkleidungsstück hat zahlreiche Vorteile. Die drei oder mehr funktional unterschiedlichen Stoffmaterialien werden gezielt an den unterschiedlichen Körperregionen im erfindungsgemäßen Beinbekleidungsstück verarbeitet und dort je nach ihrer Funktionalität wirken. Üblicherweise umfasst das Beinbekleidungsstück nicht mehr als 5 oder nicht mehr als 4 funktional unterschiedliche Stoffmaterialien. Das Beinbekleidungsstück übt mittels des ersten Stoffmaterials eine Kompression nur an jenen Stellen der Beine der Trägerin aus, wo dies auch nötig oder gewünscht ist. Diese Stellen sind bei Frauen als "Problemzonen" bekannt, wie beispielsweise die äußeren Oberschenkel und der Gesäßbereich. Durch die Kompression wird an diesen Stellen der Beine der Trägerin nicht nur eine Massage und Kompression ausgeübt, sondern die vorzugsweise erhabene Struktur des ersten Stoffmaterials, beispielsweise in Form von Noppen, wirkt zusätzlich massierend. Die Massagewirkung stellt sich insbesondre dann ein, wenn die Trägerin sich bewegt und sich somit auch die erhabene Struktur in gewissem Grad über die Haut der Trägerin bewegt und somit massierend wirkt. Darüber hinaus hat die Kompression auch eine vorteilhafte "formende" oder stützende Wirkung auf die äußeren Oberschenkel und den Gesäßbereich der Trägerin. Kleinere Dellen auf der Hautoberfläche von Oberschenkel und Gesäß, welche beispielsweise durch Cellulite verursacht werden, werden mit der erfindungsgemäßen Beinbekleidung geglättet bzw. kaschiert. Es ergibt sich im Tragezustand der erfindungsgemäßen Beinbekleidung somit nicht nur ein Massage- und Kompressionseffekt, sondern zugleich auch ein ästhetischer Effekt für die Trägerin. Aus den obigen Gründen kann das erste Stoffmaterial daher auch als Kompressionsmaterial bezeichnet werden.

Das erste Stoffmaterial bzw. das Kompressionsmaterial wird vorwiegend dazu verwendet, die großen Muskelgruppen der Beine der Trägerin abzudecken um auf diese entsprechend Druck bzw. Kompression auszuüben und diese zugleich zu stützen. Diese großen Muskelgruppen befinden sich an den äußeren Oberschenkeln, dem Gesäßbereich und den Waden der Trägerin.

An den übrigen Stellen der Beine wird über das zweite Stoffmaterial des Beinbekleidungsstücks eine geringere Kompression verglichen zum ersten Stoffmaterial ausgeübt, und über das dritte Stoffmaterial eine geringere Kompression verglichen zum zweiten Stoffmaterial oder gar keine Kompression ausgeübt. Dies sind für das zweite Stoffmaterial die Kniekehlenteile oder das Vorderteil. An diesen Stellen wird nämlich eine Kompression bzw. ein Druck als unangenehm empfunden und würde somit den Tragekomfort mindern. Insofern kann das zweite Stoffmaterial auch als Komfortmaterial bezeichnet werden. Vorzugsweise hat das zweite Stoffmaterial eine höhere Atmungsaktivität als das erste Stoffmaterial.

Das dritte Stoffmaterial wird für den Bund des erfindungsgemäßen Beinbekleidungsstück verwendet werden. Das dritte Stoffmaterial weist vorzugsweise eine samtige Oberfläche auf.

Das dritte Stoffmaterial unterscheidet sich vom zweiten Stoffmaterial in seiner Elastizität, insbesondre weist das dritte Stoffmaterial eine höhere Elastizität und/oder eine weichere oder samtigere Oberfläche auf als das zweite Stoffmaterial Das dritte Stoffmaterial weist somit den höchsten Komfort aller drei oder mehr verwendeten Stoffmaterialien des Beinbekleidungsstücks auf. Insbesondere weist das dritte Stoffmaterial einen höheren Komfort auf sowohl gegenüber dem zweiten Stoffmaterial als auch dem ersten Stoffmaterial.

Das erfindungsgemäße Beinbekleidungsstück umfasst mehrere Teile, nämlich einen Bund, ein Vorderteil, ein Rückteil, zwei Seitenteile, zwei Kniekehlenteile sowie zwei Wadenteile. Vorzugsweise besteht das erfindungsgemäße Beinbekleidungsstück aus einem Bund, einem Vorderteil, einem Rückteil, zwei Seitenteilen, zwei Kniekehlenteilen sowie zwei Wadenteilen.

Im Sinne dieser Anmeldung werden folgende Definitionen verwendet. Mit den Richtungs- bzw. Lagebezeichnungen "proximal" und "distal" wird "zum Körperzentrum hin gelegen" bzw. "vom Körperzentrum entfernt gelegen" bezeichnet. Hat ein Teil des erfindungsgemäßen Beinbekleidungsstück beispielsweise ein proximales Ende und ein distales Ende, so befindet sich im Tragezustand des Beinbekleidungsstück das proximale Ende des Teils näher am oder Richtung des Rumpfes der Trägerin, während sich das distale Ende des Teils näher am oder Richtung des Fußes der Trägerin befindet.

Der Bund des Beinbekleidungsstücks liegt im Tragezustand am unteren Bauch, an der Taille und dem unteren Rücken der Trägerin auf. Für den Bund wird das dritte Stoffmaterial, d.h. das Stoffmaterial mit höchstem Komfort, verwendet. Der Bund weist einen oberen Bundrand und einem unteren Bundrand auf. Hierbei ist der obere Bundrand proximal, der untere Bundrand distal angeordnet. Die Breite des Bundes, also der Abstand zwischen oberen Bundrand und unteren Bundrand, ist vorzugsweise zwischen 0.5 cm und 15 cm, mehr bevorzugt zwischen 2 cm und 12 cm. Der Bund des Beinbekleidungsstücks ist vorzugsweise einstückig ausgebildet, alternativ zweistückig ausgebildet. Ist der Bund zweistückig ausgebildet, so umfasst der Bund vorzugsweise ein vorderes Bundteil und ein hinteres Bundteil, wobei das vordere Bundteil mit dem hinteren Bundteil im Tragezustand des Beinbekleidungsstücks jeweils seitlich im Bereich der Hüfte und/oder Taille der Trägerin miteinander verbunden ist.

Mit vorderen Bereich des Bundes bzw. hinteren Bereich des Bundes ist jener Bereich des Bundes, der im Tragezustand des Beinbekleidungsstücks bauchseitig bzw. rückenseitig an der Trägerin zu liegen kommt, gemeint. Mit einem seitlichen Bereich des Bundes ist somit jener Bereich des Bundes, der im Tragezustand der Hose seitlich an der Trägerin zu liegen kommt, gemeint.

Einstückig im Sinne dieser Anmeldung bedeutet, dass das jeweilige Teil aus einem einzigen, durchgehenden Stück eines Stoffmaterials besteht. Zweistückig im Sinne dieser Anmeldung bedeutet, dass das jeweilige Teil aus zwei miteinander verbundenen Stücken desselben Stoffmaterials besteht. Das Verbinden kann hierbei mittels Vernähens, Vernadeln, Verkleben oder anderen im Textilbereich bekannten Techniken erfolgen, die die beiden Stücke dauerhaft miteinander verbinden.

Das dritte Stoffmaterial weist vorzugsweise eine samtige Oberfläche auf, d.h. das dritte Stoffmaterial umfasst oder besteht aus einem Gewebe mit einer Haardecke von emporstehenden Fäden oder Fasern, die eine üblicherweise 1 bis 3 mm hohe Flordecke bilden. Samt ist im Stand der Technik bekannt. Das dritte Stoffmaterial wird vorzugsweise verstürzt als Bund eingesetzt. Das dritte Stoffmaterial umfasst vorzugsweise Stretchmaterial, vorzugsweise Multiway-Stretchmaterial, oder Moleskin. Ein Multiway-Stretchmaterial lässt sich in alle Richtungen leicht dehnen. Moleskin ist ein Baumwollstoff und auch unter dem Namen Englischleder bekannt.

Das Beinbekleidungsstück umfasst ferner zwei Seitenteile, nämlich ein linkes Seitenteil und ein rechtes Seitenteil, wobei sowohl das linke als auch das rechte Seitenteil jeweils ein proximales Ende und ein distales Ende aufweisen. Die beiden Seitenteile sind, jeweils mit ihrem proximalen Ende, mit dem Bund verbunden, vorzugsweise mit dem unteren Bundrand des Bundes. Im getragenen Zustand des Beinbekleidungsstücks kommt das linke Seitenteil am linken Bein der Trägerin, und entsprechend das rechte Seitenteil am rechten Bein der Trägerin zu liegen, und bedecken damit die Muskelgruppen an den äußeren Oberschenkeln der Trägerin.

Vorzugsweise erstrecken sich die zwei Seitenteile im getragenen Zustand des Beinbekleidungsstücks jeweils ausgehend von einem seitlichen Bereich des Bund über den äußeren Oberschenkelbereich bis zu einem äußeren seitlichen Kniebereich der Trägerin, mehr bevorzugt jeweils ausgehend von einem seitlichen Bereich des unteren Bundrands über den äußeren Oberschenkelbereich bis zumindest zu einem seitlichen Kniebereich der Trägerin. Hierbei sind die beiden Seitenteile mit ihren jeweiligen distalen Enden vorzugsweise mit den jeweiligen Vorderteilen verbunden, mehr bevorzugt mit den jeweiligen Vorderteilen und zugleich mit den jeweiligen Kniekehlenteilen verbunden.

Vorzugsweise weisen die zwei Seitenteile jeweils eine bandförmige Gestalt auf und/oder sind die zwei Seitenteile jeweils einstückig ausgebildet.

Das Beinbekleidungsstück weist ferner ein Vorderteil auf, wobei das Vorderteil ein proximales Ende und ein distales Ende aufweist. Für das Vorderteil wird das Komfortmaterial, d.h. das zweite Stoffmaterial, verwendet.

Vorzugsweise erstreckt sich das Vorderteil im getragenen Zustand des Beinbekleidungsstücks ausgehend von einem vorderen Bereich des Bundes, vorzugsweise von einem vorderen Bereich des unteren Bundrands, über den Schrittbereich, die beiden vorderen Oberschenkelbereiche und Innenschenkelbereich bis zumindest jeweils zu einem vorderen Kniebereich der Trägerin, mehr bevorzugt bis zumindest jeweils zu einem Knöchelbereich der Trägerin. Ist der Bund zweistückig ausgebildet, so erstreckt sich vorzugsweise das Vorderteil im getragenen Zustand ausgehend von einem vorderen Bundteil, mehr bevorzugt von einem unteren Rand des vorderen Bundteils, über die beiden vorderen Oberschenkelbereiche bis zumindest jeweils zu einem vorderen Kniebereich der Trägerin, mehr bevorzugt bis zumindest jeweils zu einem Knöchelbereich der Trägerin.

Das Vorderteil kann einstückig oder zweistückig ausgebildet sein. Ist das Vorderteil zweistückig ausgebildet, so umfasst das Vorderteil ein linkes Vorderteil und ein rechtes Vorderteil, welche zueinander symmetrisch ausgebildet sind. Das linke und das rechte Vorderteil sind entlang einer medialen Linie, welche von einem Schrittbereich des Beinbekleidungsstücks bis zum unteren Bundrand, vorzugsweise bis zum unteren Bundrand des vorderen Bundteils, verläuft, miteinander verbunden. Die Verbindung kann mittels Vernähens, Vernadeln, Verkleben oder anderen im Textilbereich bekannten Techniken erfolgen, die die beiden Stücke dauerhaft miteinander verbinden.

Das Beinbekleidungsstück weist ferner ein Rückteil auf. Das Rückteil ist vorzugsweise einstückig ausgebildet, kann aber auch zweistückig ausgebildet sein. Für das Rückteil wird das Kompressionsmaterial, d.h. das erste Stoffmaterial, zwecks Kompression der Gesäßmuskeln der Trägerin verwendet.

Vorzugsweise erstreckt sich das Rückteil im getragenen Zustand des Beinbekleidungsstücks ausgehend von einem hinteren Bereich des Bundes, vorzugsweise von einem hinteren Bereich des unteren Bundrands, über einen Gesäßbereich und die beiden hinteren Oberschenkelbereiche der Trägerin bis zu den jeweiligen Kniekehlenteilen des Beinbekleidungsstücks. Vorzugsweise ist das Rückenteil mit den beiden Kniekehlenteilen verbunden. Mit anderen Worten, die jeweiligen distalen Enden des Rückteils erstrecken sich bis zu den proximalen Enden der jeweiligen Kniekehlenteilen und sind mit diesen verbunden. Die Verbindung kann mittels Vernähens, Vernadeln, Verkleben oder anderen im Textilbereich bekannten Techniken erfolgen, die die beiden Teile dauerhaft miteinander verbinden.

Das Rückteil kann einstückig oder zweistückig ausgebildet sein. Ist das Rückteil zweistückig ausgebildet, so umfasst das Rückteil ein linkes Rückteil und ein rechtes Rückteil, welche zueinander symmetrisch ausgebildet sind. Das linke und das rechte Rückteil sind entlang einer medialen Linie, welche von einem Schrittbereich des Beinbekleidungsstücks bis zum unteren Bundrand, vorzugsweise bis zum unteren Bundrand des hinteren Bundteils, verläuft, miteinander verbunden. Die Verbindung kann mittels Vernähens, Vernadeln, Verkleben oder anderen im Textilbereich bekannten Techniken erfolgen, die die beiden Stücke dauerhaft miteinander verbinden.

Das Beinbekleidungsstück weist zudem zwei Kniekehlenteile auf, nämlich ein linkes Kniekehlenteil und ein rechtes Kniekehlenteil, wobei sowohl das linke als auch das rechte Kniekehlenteil jeweils ein proximales Ende und ein distales Ende aufweisen. Das linke und das rechte Kniekehlenteil sind vorzugsweise zueinander symmetrisch ausgebildet. Für die Kniekehlenteile wird das Komfortmaterial, d.h. das zweite Stoffmaterial, verwendet.

Im getragenen Zustand des Beinbekleidungsstücks kommt das linke Kniekehlenteil im Bereich der Kniekehle des linken Beins der Trägerin, und entsprechend das rechte Kniekehlenteil getragenen Zustand des Beinbekleidungsstücks kommt das linke Kniekehlenteil im Bereich der Kniekehle des rechten Beins der Trägerin zu liegen. Vorzugsweise erstrecken sich die beiden Kniekehlenteile jeweils auch über die unteren, hinteren Oberschenkelbereiche der Trägerin und/oder erstrecken sich die beiden Kniekehlenteile jeweils auch über die oberen Wadenbereiche der Trägerin.

Die Kniekehlenteile sind vorzugsweise jeweils an ihrem proximalen Ende mit dem distalen Ende des Rückteils verbunden, und jeweils mit ihrem distalen Ende mit den jeweiligen Wadenteil verbunden. Die Verbindung kann mittels Vernähens, Vernadeln, Verkleben oder anderen im Textilbereich bekannten Techniken erfolgen, die die beiden Stücke dauerhaft miteinander verbinden.

Das Beinbekleidungsstück weist zudem zwei Wadenteile auf, nämlich ein linkes Wadenteil und ein rechtes Wadenteil, wobei sowohl das linke als auch das rechte Wadenteil jeweils ein proximales Ende und ein distales Ende aufweisen. Für das Wadenteil wird das Kompressionsmaterial, d.h. das erste Stoffmaterial, zwecks Kompression der Wadenmuskeln der Trägerin verwendet.

Im getragenen Zustand des Beinbekleidungsstücks kommt das linke Wadenteil im Wadenbereich des linken Beins der Trägerin, und entsprechend das rechte Wadenteil im Wadenbereich des rechten Beins der Trägerin zu liegen. Somit werden mit den Wadenteilen die jeweiligen Wadenmuskeln der Trägerin bedeckt und komprimiert.

Die Wadenteile sind vorzugsweise jeweils an ihrem proximalen Ende mit dem distalen Ende des jeweiligen Kniekehlenteils verbunden. Die Verbindung kann mittels Vernähens, Vernadeln, Verkleben oder anderen im Textilbereich bekannten Techniken erfolgen, die die beiden Stücke dauerhaft miteinander verbinden.

Vorzugsweise weist das erste Stoffmaterial eine erhabene Struktur auf, und die erhabene Struktur des ersten Stoffmaterials steht im getragenen Zustand in direktem Kontakt mit der Haut der Trägerin des Beinbekleidungsstücks und/oder weist das zweite Stoffmaterial eine glatte Struktur auf, und die glatte Struktur des zweiten Stoffmaterials steht im getragenen Zustand in direktem Kontakt zur Haut der Trägerin des Beinbekleidungsstücks. Mit direktem Kontakt ist gemeint, dass das jeweilige Stoffmaterial direkt auf der Haut der Trägerin aufliegt, d.h. es befindet sich keine Stofflage oder ähnliches zwischen der Haut der Trägerin und des jeweiligen Stoffmaterials.

Vorzugsweise unterscheidet sich das erste Stoffmaterial, vorzugsweise mit erhabener Struktur, in der Kompression, in der Dehnung, im Gewicht, in den Fasereigenschaften und/oder den Garneigenschaften von dem zweiten Stoffmaterial. Insbesondere weist das erste Stoffmaterial eine höhere Kompression und/oder eine geringere Dehnung als das zweite Stoffmaterial auf.

Vorzugsweise umfasst das erste Stoffmaterial, d.h. das Kompressionsmaterial, Polyamid, Viskose oder Polyester, mehr bevorzugt eine Kombination aus Polyamid mit 1-30% Elasthan oder eine Kombination aus Polyester mit 1-30% Elasthan. Der Elasthananteil kann entsprechend den Bedürfnissen hinsichtlich Elastizität und Kompressionseigenschaften des ersten Stoffmaterials im genannten Bereich angepasst werden.

Vorzugsweise umfasst das zweite Stoffmaterial, d.h. das Komfortmaterial, Baumwolle oder Stretchmaterial, mehr bevorzugt umfasst das zweite Stoffmaterial Pikee oder ein Multiway-Stretchmaterial. Baumwolle, insbesondere als Pikee, ist atmungsaktiv und eignet sich daher besonders als Komfortmaterial. Ein Multiway-Stretchmaterial lässt sich in alle Richtungen leicht dehnen.

Vorzugsweise ist die erhabene Struktur des ersten Stoffmaterials in Form von Noppen ausgebildet. Dabei sind die Noppen vorzugsweise in einem regelmäßigen oder einem unregelmäßigen Muster auf dem Stoffmaterial angeordnet. Die Noppen können jede beliebige Form annehmen, beispielsweise kreisförmige Noppen sowie drei-, vier- oder vieleckige Noppen. Die Noppen können gestrickt oder gewirkt sein.

Vorzugsweise erstreckt sich jedes Wadenteil im getragenen Zustand des Beinbekleidungsstücks vom jeweiligen Kniekehlenteil bis zu einem Hosenbeinabschluss.

Vorzugsweise bilden das Vorderteil und das jeweilige Wadenteil jeweils einen gemeinsamen Hosenbeinabschluss, mehr bevorzugt bilden die beiden distalen Enden des Vorderteils mit den jeweiligen distalen Enden der beiden Wadenteile einen gemeinsamen Hosenbeinabschluss des Beinbekleidungsstücks.

Vorzugsweise bilden das Vorderteil, das jeweilige Kniekehlenteil und das jeweilige Wadenteil jeweils einen gemeinsamen Hosenbeinabschluss, mehr bevorzugt bilden die beiden distalen Enden des Vorderteils mit den jeweiligen distalen Enden der beiden Kniekehlenteile und mit den jeweiligen distalen Enden der beiden Wadenteile einen gemeinsamen Hosenbeinabschluss des Beinbekleidungsstücks.

Vorzugsweise ist das Beinbekleidungsstück eine Leggins oder eine Hose. Die Hose ist vorzugsweise eine Laufhose oder eine Gymnastikhose. Die Hose, insbesondre eine Laufhose oder eine Gymnastikhose, kann eine kurze, eine halblange oder eine lange Hose, insbesondere eine Laufhose oder eine Gymnastikhose, sein.

Vorzugsweise befindet sich der Hosenbeinabschluss im Bereich der Kniekehle der Trägerin. In diesem Fall ist die Hose als halblange Hose ausgeführt.

Vorzugsweise befindet sich der Hosenbeinabschluss im Bereich der Wade der Trägerin. In diesem Fall ist die Hose als halblange Hose ausgeführt.

Vorzugsweise befindet sich der Hosenbeinabschluss im Bereich des Knöchels der Trägerin. In diesem Fall ist die Hose als lange Hose ausgeführt.

Nachstehend ist die Erfindung anhand der beigefügten Figuren beispielhaft näher erläutert. Darin zeigt
Figur 1 eine Seitenansicht des Beinbekleidungsstückes gemäß einer Ausführungsform der Erfindung,
Figur 2 eine Vorderansicht des Beinbekleidungsstückes gemäß der Ausführungsform von Figur 1,
Figur 3 eine Rückansicht des Beinbekleidungsstückes gemäß der Ausführungsform von Figur 1,

### Referenzzeichen

- 1: Beinbekleidungsstück
- 2: Bund
- 2a: vorderes Bundteil
- 2b: hinteres Bundteil
- 3: Vorderteil
- 3a: rechtes Vorderteil
- 3b: linkes Vorderteil
- 4a: rechtes Seitenteil
- 4b: linkes Seitenteil
- 5: Rückteil
- 5a: Rechtes Rückteil
- 5b: Linkes Rückteil
- 6a: Rechtes Kniekehlenteil
- 6b: Linkes Kniekehlenteil
- 7a: Rechtes Wadenteil
- 7b: Linkes Wadenteil
- 8: Hosenbeinabschluss

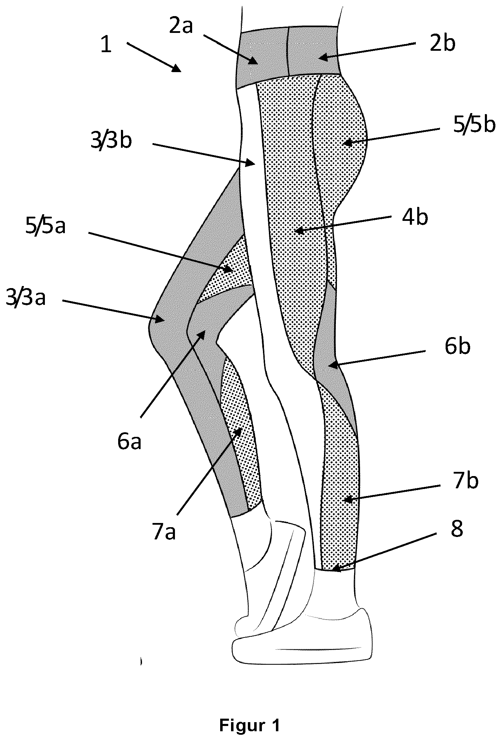

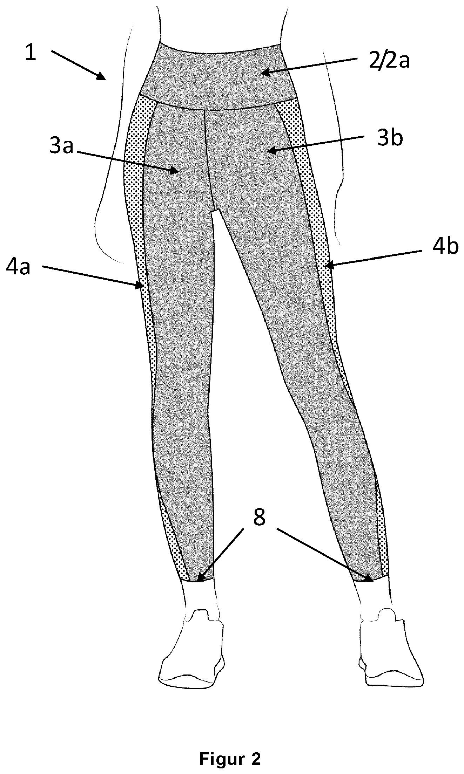

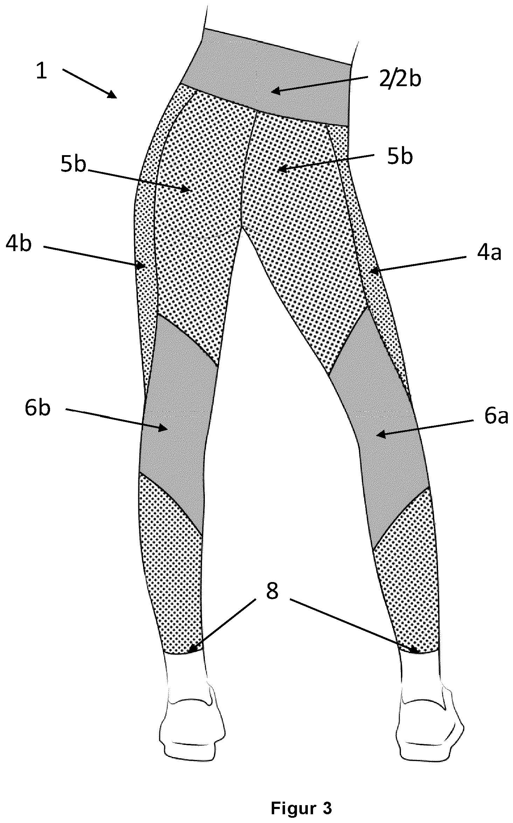

Das Beinbekleidungsstück (1) der in den Figuren 1 bis 3 gezeigten beispielhaften Ausführungsform der Erfindung ist in Form hautenger Leggins ausgeführt. Es weist einen zweistückigen Bund (2) mit einem vorderen Bundteil (2a) und einem hinteren Bundteil (2b) auf. Wie aus Figur 1 ersichtlich ist der vordere Bundteil (2a) seitlich im Bereich der Hüfte der Trägerin mit dem hinteren Bundteil (2b) vernäht. Der Bund (2) ist bandförmig ausgestaltet und kommt im Tragezustand im Taillen- und Hüftbereich der Trägerin zu liegen. Die Bundbreite, d.h. der Abstand zwischen einem oberen und einem unteren Bundrand des Bunds (2), beträgt etwa 10 cm.

Das Beinbekleidungsstück (1) umfasst ferner ein Vorderteil (3) welches hier als zweistückiges Vorderteil ausgestaltet ist, nämlich einem rechten Vorderteil (3a) und einem linken Vorderteil (3b) welche zueinander symmetrisch ausgebildet sind. Das Vorderteil (3) erstreckt sich ausgehend vom unteren Bundrand des Bundes (2) über die vorderen Oberschenkelbereiche und die vorderen Kniebereich bis hin zu einem Fußknöchelbereich der Trägerin.

Das Beinbekleidungsstück umfasst ferner zwei Seitenteile, nämlich einem rechten Seitenteil (4a) und einem linken Seitenteil (4b) welche ebenfalls zueinander symmetrisch ausgebildet sind. Die beiden Seitenteile erstrecken sich jeweils vom unteren Bundrand des Bundes (2) über die seitlichen Oberschenkel bis zu einem seitlichen Kniebereich der Trägerin.

Ferner weist das Beinbekleidungsstück (1) ein Rückteil (5) auf. Das Rückteil (5) ist zweistückig ausgebildet, und umfasst ein linkes Rückteil (5b) und ein rechtes Rückteil (5a). Beide Rückteile erstrecken sich jeweils vom unteren Bundrand des Bundes (2) über da Gesäß der Trägerin bis nahe den Kniekehlen der Trägerin. Die distalen Enden der Rückteile sind dort mit den proximalen Enden der jeweiligen Kniekehlenteile (6a, 6b) vernäht. Die distalen Enden der Kniekehlenteile (6a, 6b) sind mit den proximalen Enden der jeweiligen Wadenteile (7a, 7b) vernäht.

Gemeinsam mit den zwei Wadenteilen (7a, 7b) bilden die jeweiligen Vorderteile (3a, 4b) die beiden Hosenbeinabschlüsse (8).

Das zweistückige Vorderteil (3a, 3b), und die beiden Kniekehlenteile (6a, 6b) bestehen aus Pikee, d.h. dem zweiten Stoffmaterial. Die gesteppte Oberfläche des Pikee ist nach außer angeordnet, während die glatte Oberfläche des Pikee zur Haut der Trägerin zugewandt ist.

Der Bund (2) besteht aus einem dritten Stoffmaterial, welches in diesem Fall ein Multiway-Stretchmaterial mit samtiger Oberfläche ist.

Im Gegensatz dazu bestehen die beiden Seitenteile (4a, 4b), die beiden Rückteile (5a, 5b) sowie die beiden Wadenteile (7a, 7b) aus dem ersten Stoffmaterial, d.h. einem Kompressionsmaterial. Das erste Stoffmaterial ist in diesem Falle eine Mischung aus Polyamid und Elasthan.

## Patentansprüche

1. Beinbekleidungsstück (1) aus drei oder mehr unterschiedlichen Stoffmaterialien, umfassend einen Bund (2), ein Vorderteil (3), ein Rückteil (5), zwei Seitenteile (4a, 4b), zwei Kniekehlenteile (6a, 6b) sowie zwei Wadenteile (7a, 7b), wobei das Vorderteil (3), die Seitenteile (4a, 4b) und das Rückteil (5) jeweils mit dem Bund (2) verbunden sind, **dadurch gekennzeichnet, dass**
die zwei Seitenteile (4a, 4b), das Rückteil (5) und die zwei Wadenteile (7a, 7b) jeweils ein erstes Stoffmaterial zur Massage und Kompression von Beinbereichen einer Trägerin umfassen, dass
das Vorderteil (3) und die Kniekehlenteile (6a, 6b) jeweils ein zweites Stoffmaterial umfasst, und dass
der Bund (2) ein drittes Stoffmaterial umfasst,
wobei das erste Stoffmaterial eine höhere Kompression aufweist als das zweite Stoffmaterial.

2. Beinbekleidungsstück (1) nach einem der vorhergehenden Ansprüche, **dadurch gekennzeichnet, dass** das erste Stoffmaterial eine erhabene Struktur aufweist, und die erhabene Struktur des ersten Stoffmaterials im getragenen Zustand in direktem Kontakt mit der Haut der Trägerin des Beinbekleidungsstücks (1) steht und/oder dass das zweite Stoffmaterial eine glatte Struktur aufweist, und die glatte Struktur des zweiten Stoffmaterials im getragenen Zustand in direktem Kontakt zur Haut der Trägerin des Beinbekleidungsstücks (1) steht.

3. Beinbekleidungsstück (1) nach einem der vorhergehenden Ansprüche, **dadurch gekennzeichnet, dass** das erste Stoffmaterial Polyamid, Viskose oder Polyester umfasst und/oder dass das zweite Stoffmaterial Baumwolle oder Stretchmaterial, vorzugsweise Multiway-Stretchmaterial, umfasst.

4. Beinbekleidungsstück (1) nach einem der vorhergehenden Ansprüche, **dadurch gekennzeichnet, dass** das zweite Stoffmaterial eine höhere Atmungsaktivität als das erste Stoffmaterial hat.

5. Beinbekleidungsstück (1) nach einem der vorhergehenden Ansprüche, **dadurch gekennzeichnet, dass** das dritte Stoffmaterial eine samtige Oberfläche aufweist.

6. Beinbekleidungsstück (1) nach einem der vorhergehenden Ansprüche, **dadurch gekennzeichnet, dass** das erste Stoffmaterial, das zweite Stoffmaterial und das dritte Stoffmaterial sich in ihren Kompressionseigenschaften, ihren Dehnungseigenschaften, ihren Quadratmetergewicht, ihrer Luftdurchlässigkeit, ihrer Oberflächenstruktur, ihren Fasereigenschaften und/oder den Garneigenschaften voneinander unterscheiden.

7. Beinbekleidungsstück (1) nach einem der Ansprüche 2 bis 6, **dadurch gekennzeichnet, dass** die erhabene Struktur des ersten Stoffmaterials in Form von Noppen ausgebildet ist.

8. Beinbekleidungsstück (1) nach einem der vorhergehenden Ansprüche, **dadurch gekennzeichnet, dass** sich das Vorderteil (3) im getragenen Zustand ausgehend von einem vorderen Bereich des Bundes (2) über den Schrittbereich, die beiden vorderen Oberschenkelbereiche und Innenschenkelbereiche bis zumindest jeweils zu einem vorderen Kniebereich der Trägerin erstreckt.

9. Beinbekleidungsstück (1) nach einem der vorhergehenden Ansprüche, **dadurch gekennzeichnet, dass** sich das Rückteil (5) im getragenen Zustand ausgehend von einem hinteren Bereich des Bundes (2) über einen Gesäßbereich und die beiden hinteren Oberschenkelbereiche der Trägerin bis zu den jeweiligen Kniekehlenteilen (6a, 6b) erstreckt.

10. Beinbekleidungsstück (1) nach einem der vorhergehenden Ansprüche, **dadurch gekennzeichnet, dass** sich die Seitenteile (4a, 4b) im getragenen Zustand ausgehend von einem seitlichen Bereich des Bundes (2) über den äußeren Oberschenkelbereich bis zu einem äußeren seitlichen Kniebereich der Trägerin erstrecken.

11. Beinbekleidungsstück (1) nach einem der vorhergehenden Ansprüche, **dadurch gekennzeichnet, dass** die zwei Seitenteile (4a, 4b) jeweils eine bandförmige Gestalt aufweisen.

12. Beinbekleidungsstück (1) nach einem der vorhergehenden Ansprüche, **dadurch gekennzeichnet, dass** die zwei Seitenteile (4a, 4b) jeweils einstückig ausgebildet sind.

13. Beinbekleidungsstück (1) nach einem der vorhergehenden Ansprüche, **dadurch gekennzeichnet, dass** das Vorderteil (3a, 3b) zweistückig ausgebildet ist und/oder dass das Rückteil (5a, 5b) zweistückig ausgebildet ist.

14. Beinbekleidungsstück (1) nach einem der vorhergehenden Ansprüche, **dadurch gekennzeichnet, dass** das Vorderteil (3) und das jeweilige Wadenteil (7a, 7b) jeweils einen gemeinsamen Hosenbeinabschluss (8) bilden, oder dass das Vorderteil (3), das jeweilige Kniekehlenteil (6a, 6b) und das jeweilige Wadenteil (7a, 7b) jeweils einen gemeinsamen Hosenbeinabschluss (8) bilden.

15. Beinbekleidungsstück (1) nach Anspruch 14, **dadurch gekennzeichnet, dass** sich der Hosenbeinabschluss (8) im Bereich der Wade der Trägerin befindet oder dass sich der Hosenbeinabschluss (8) im Bereich des Knöchels der Trägerin befindet.

## Claims

1. A legwear item (1) made of three or more different cloth materials and comprising a waistband (2), a front panel (3), a rear panel (5), two side panels (4a, 4b), two knee hollow panels (6a, 6b), and two calf panels (7a, 7b), the front panel (3), the side panels (4a, 4b), and the rear panel (5) each being connected to the waistband (2), **characterized in that**
the two side panels (4a, 4b), the rear panel (5), and the two calf panels (7a, 7b) each comprise a first cloth material for massaging and compressing leg regions of a wearer, and **in that**
the front panel (3) and the knee hollow panels (6a, 6b) each comprise a second cloth material, and **in that**
the waistband (2) comprises a third cloth material,
wherein the first cloth material has a greater compression than the second cloth material.

2. The legwear item (1) according to any one of the preceding claims, **characterized in that** the first cloth material comprises a raised structure and the raised structure of the first cloth material directly contacts the skin of the wearer of the legwear item (1) in the worn state, and/or **in that** the second cloth material comprises a smooth structure, and the smooth structure of the second cloth material directly contacts the skin of the wearer of the legwear item (1) in the worn state.

3. The legwear item (1) according to any one of the preceding claims, **characterized in that** the first cloth material comprises polyamide, viscose, or polyester, and/or **in that** the second cloth material comprises cotton or stretch material, preferably multiway stretch material.

4. The legwear item (1) according to any one of the preceding claims, **characterized in that** the second cloth material has greater breathability than the first cloth material.

5. The legwear item (1) according to any one of the preceding claims, **characterized in that** the third cloth material comprises a velvety surface.

6. The legwear item (1) according to any one of the preceding claims, **characterized in that** the first cloth material, the second cloth material, and the third cloth material differ from each other in the compression properties, stretching properties, grammage, air-permeability, surface structure, fiber properties, and/or yarn properties thereof.

7. The legwear item (1) according to any one of the claims 2 through 6, **characterized in that** the raised structure of the first cloth material is implemented in the form of napping.

8. The legwear item (1) according to any one of the preceding claims, **characterized in that** the front panel (3) extends in the worn state from a front region of the waistband (2), across the crotch region, the two front thigh regions and inner thigh regions, to at least one respective front knee region of the wearer.

9. The legwear item (1) according to any one of the preceding claims, **characterized in that** the rear panel (5) extends in the worn state from a rear region of the waistband (2), across a buttocks region and the two rear thigh regions of the wearer, to the corresponding knee hollow panels (6a, 6b).

10. The legwear item (1) according to any one of the preceding claims, **characterized in that** the side panels (4a, 4b) extend in the worn state from a side region of the waistband (2), across the outer thigh region, to an outer side knee region of the wearer.

11. The legwear item (1) according to any one of the preceding claims, **characterized in that** the two side panels (4a, 4b) each have a band-shaped design.

12. The legwear item (1) according to any one of the preceding claims, **characterized in that** the two side panels (4a, 4b) are each implemented as a single piece.

13. The legwear item (1) according to any one of the preceding claims, **characterized in that** the front panel (3a, 3b) is implemented in two pieces and/or **in that** the rear panel (5a, 5b) is implemented in two pieces.

14. The legwear item (1) according to any one of the preceding claims, **characterized in that** the front panel (3) and the corresponding calf panel (7a, 7b) each form a common trouser leg hem (8), or **in that** the front panel (3), the corresponding knee hollow panel (6a, 6b), and the corresponding calf panel (7a, 7b) each form a common trouser leg hem (8).

15. The legwear item (1) according to claim 14, **characterized in that** the trouser leg hem (8) is present in the region of the calf of the wearer, or **in that** the trouser leg hem (8) is present in the region of the ankle of the wearer.

## Revendications

1. Vêtement de jambe (1) en trois ou plus de trois matériaux de tissu différents, comprenant une ceinture (2), une partie avant (3), une partie arrière (5), deux parties latérales (4a, 4b), deux parties de creux de genou (6a, 6b) ainsi que deux parties de mollet (7a, 7b), la partie avant (3), les parties latérales (4a, 4b) et la partie arrière (5) étant respectivement reliées à la ceinture (2),
**caractérisé en ce que**
les deux parties latérales (4a, 4b), la partie arrière (5) et les deux parties de mollet (7a, 7b) comprennent chacune un premier matériau de tissu pour le massage et la compression de zones de jambes d'une utilisatrice, **en ce que** la partie avant (3) et les parties de creux genou (6a, 6b) comprennent chacune un deuxième matériau de tissu, et **en ce que**
la ceinture (2) comprend un troisième matériau de tissu,
le premier matériau de tissu présentant une compression plus élevée que le deuxième matériau de tissu.

2. Vêtement de jambe (1) selon l'une quelconque des revendications précédentes, **caractérisé en ce que** le premier matériau de tissu présente une structure en relief, et la structure en relief du premier matériau de tissu, lorsqu'il est porté, est en contact direct avec la peau de l'utilisatrice du vêtement de jambe (1) et/ou **en ce que** le deuxième matériau de tissu présente une structure lisse, et la structure lisse du deuxième matériau de tissu, lorsqu'il est porté, est en contact direct avec la peau de l'utilisatrice du vêtement de jambe (1).

3. Vêtement de jambe (1) selon l'une quelconque des revendications précédentes, **caractérisé en ce que** le premier matériau de tissu comprend du polyamide, de la viscose ou du polyester et/ou **en ce que** le deuxième matériau de tissu comprend du coton ou un matériau stretch, de préférence un matériau stretch multidirectionnel.

4. Vêtement de jambe (1) selon l'une quelconque des revendications précédentes, **caractérisé en ce que** le deuxième matériau de tissu a une respirabilité supérieure à celle du premier matériau de tissu.

5. Vêtement de jambe (1) selon l'une quelconque des revendications précédentes, **caractérisé en ce que** le troisième matériau de tissu présente une surface veloutée.

6. Vêtement de jambe (1) selon l'une quelconque des revendications précédentes,
**caractérisé en ce que** le premier matériau de tissu, le deuxième matériau de tissu et le troisième matériau de tissu diffèrent les uns des autres dans leurs propriétés de compression, leurs propriétés d'étirement, leur poids au mètre carré, leur perméabilité à l'air, leur
structure de surface, les propriétés de leurs fibres et/ou les propriétés de leurs fils.

7. Vêtement de jambe (1) selon l'une quelconque des revendications 2 à 6, **caractérisé en ce que** la structure en relief du premier matériau de tissu est réalisée sous la forme de picots.

8. Vêtement de jambe (1) selon l'une quelconque des revendications précédentes, **caractérisé en ce que** la partie avant (3) s'étend, à l'état porté, à partir d'une zone avant de la ceinture (2), en passant par la zone d'entrejambe, les deux zones avant des cuisses et les zones intérieures des cuisses, jusqu'à au moins respectivement une zone avant des genoux de l'utilisatrice.

9. Vêtement de jambe (1) selon l'une quelconque des revendications précédentes, **caractérisé en ce que** la partie arrière (5) s'étend, à l'état porté, depuis une zone arrière de la ceinture (2) jusqu'aux parties respectives du creux du genou (6a, 6b), en passant par une zone des fesses et les deux zones arrière des cuisses de l'utilisatrice.

10. Vêtement de jambe (1) selon l'une quelconque des revendications précédentes, **caractérisé en ce que** les parties latérales (4a, 4b) s'étendent, à l'état porté, depuis une zone latérale de la ceinture (2) jusqu'à une zone latérale extérieure du genou de l'utilisatrice, en passant par la zone extérieure de la cuisse.

11. Vêtement de jambe (1) selon l'une quelconque des revendications précédentes, **caractérisé en ce que** les deux parties latérales (4a, 4b) présentent chacune une forme de bande.

12. Vêtement de jambe (1) selon l'une quelconque des revendications précédentes, **caractérisé en ce que** les deux parties latérales (4a, 4b) sont chacune formées d'une seule pièce.

13. Vêtement de jambe (1) selon l'une quelconque des revendications précédentes, **caractérisé en ce que** la partie avant (3a, 3b) est réalisée en deux pièces et/ou **en ce que** la partie arrière (5a, 5b) est réalisée en deux pièces.

14. Vêtement de jambe (1) selon l'une quelconque des revendications précédentes, **caractérisé en ce que** la partie avant (3) et la partie de mollet respective (7a, 7b) forment chacune une extrémité de jambe de pantalon commune (8), ou **en ce que** la partie avant (3), la partie de creux de genou respective (6a, 6b) et la partie de mollet respective (7a, 7b) forment chacune une extrémité de jambe de pantalon commune (8).

15. Vêtement de jambe (1) selon la revendication 14, **caractérisé en ce que** l'extrémité de la jambe de pantalon (8) se trouve dans la zone du mollet de l'utilisatrice ou **en ce que** l'extrémité de la jambe de pantalon (8) se trouve dans la zone de la cheville de l'utilisatrice.
